# EUROPÄISCHE PATENTANMELDUNG

(11) **EP 4 809 968 A1**
(43) Veröffentlichungstag der Anmeldung: **23.09.2026**
(21) Anmeldenummer: 25165438.0
(22) Anmeldetag: 21.03.2025
(51) Int. Cl.: A61C 7/00, A61C 7/08

(54) **VERFAHREN ZUM BESTIMMEN EINER SOLL-AUSSENFLÄCHE EINER ZAHNSCHIENE, VERFAHREN ZUM HERSTELLEN EINER ZAHNSCHIENE, ZAHNSCHIENE, VORRICHTUNG ZUR DATENVERARBEITUNG, COMPUTERPROGRAMM UND COMPUTERLESBARES MEDIUM**

(71) Anmelder: Ivoclar Vivadent AG, 9494 Schaan (LI)
(72) Erfinder: SKAWRAN, Alexander, 7320 Sargans (CH); SCHÜLKE, Andreas, 9472 Grabs (CH)
(74) Vertreter: Maiwald GmbH

(57) **Zusammenfassung**

Es wird ein computer-implementiertes Verfahren zum Bestimmen einer Soll-Außenfläche (46) einer Zahnschiene beschrieben. Dabei wird ein virtuelles Zahnschienenrohlingmodell (30) basierend auf einem virtuellen Zahnmodell (28) erzeugt. Ferner wird das virtuelle Zahnschienenrohlingmodell (30) basierend auf Materialdaten erzeugt, die wenigstens eine Eigenschaft eines Materials beschreiben, aus dem die Zahnschiene hergestellt werden soll. Alternativ wird das virtuelle Zahnschienenrohlingmodell (30) erhalten. Zusätzlich wird ein virtuelles Antagonistenmodell (42) erhalten. Außerdem wird die Soll-Außenfläche (46) der Zahnschiene basierend auf dem virtuellen Zahnschienenrohlingmodell (30) und dem virtuellen Antagonistenmodell (42) bestimmt. Auch wird ein Verfahren zum Herstellen einer Zahnschiene sowie eine damit erhaltene Zahnschiene vorgestellt. Zudem werden eine Vorrichtung zur Datenverarbeitung, ein Computerprogramm und ein computerlesbares Medium präsentiert.

## Beschreibung

Die Erfindung ist auf ein computer-implementiertes Verfahren zum Bestimmen einer Soll-Außenfläche einer Zahnschiene gerichtet.

Auch betrifft die Erfindung ein Verfahren zum Herstellen einer Zahnschiene.

Die Erfindung ist ferner auf eine Zahnschiene gerichtet.

Zusätzlich betrifft die Erfindung eine Vorrichtung zur Datenverarbeitung, ein Computerprogramm und ein computerlesbares Medium.

Zahnschienen werden für verschiedene zahnmedizinische Behandlungen verwendet. Beispielsweise werden Zahnschienen dazu verwendet, Zahnstellungen zu korrigieren. In diesem Zusammenhang werden die Zahnschienen auch als Aligner-Schienen bezeichnet. Ein weiterer Anwendungsfall von Zahnschienen ist der Schutz der Zähne und die Entlastung der Kiefergelenke. Umgangssprachlich werden in diesem Zusammenhang die Zahnschienen auch als Knirscher-Schienen bezeichnet. Auch werden Zahnschienen zum Schutz der Zähne bei verschiedenen sportlichen Aktivitäten eingesetzt.

Bei der Herstellung von Zahnschienen muss stets berücksichtigt werden, dass einerseits die Herstellung einfach, zuverlässig und effizient sein soll. Andererseits muss die zahnmedizinische Funktion sichergestellt sein, ohne unerwünschte Nebeneffekte zu generieren. Dies birgt einen gewissen Zielkonflikt.

### SE:TOP

Die der vorliegenden Erfindung zugrundeliegende Aufgabe besteht somit darin, diesen Zielkonflikt aufzulösen oder zumindest abzumildern, indem Zahnschienen und Verfahren zu deren Herstellung derart verbessert werden, dass Zahnschienen einerseits einfach, zuverlässig und effizient hergestellt werden können und andererseits die zugeordnete zahnmedizinische Funktion ohne unerwünschte Nebeneffekte oder mit nur geringen Nebeneffekten bereitstellen können.

Die Aufgabe wird durch ein computer-implementiertes Verfahren zum Bestimmen einer Soll-Außenfläche einer Zahnschiene gelöst. Das Verfahren umfasst:
- Erzeugen eines virtuellen Zahnschienenrohlingmodells basierend auf einem virtuellen Zahnmodell, welches zumindest virtuelle Teil-Modelle derjenigen Zähne umfasst, über die sich die Zahnschiene erstrecken soll, und basierend auf Materialdaten, die wenigstens eine Eigenschaft eines Materials beschreiben, aus dem die Zahnschiene hergestellt werden soll, oder
   Erhalten eines virtuellen Zahnschienenrohlingmodells,
- Erhalten eines virtuellen Antagonistenmodells, welches zumindest virtuelle Teil-Modelle derjenigen Zähne umfasst, die den Zähnen gegenüberliegen, über die sich die Zahnschiene erstrecken soll, und
- Bestimmen der Soll-Außenfläche der Zahnschiene basierend auf dem virtuellen Zahnschienenrohlingmodell und dem virtuellen Antagonistenmodell.

Das Verfahren umfasst zwei Alternativen. Gemäß einer ersten Alternative wird das virtuelle Zahnschienenrohlingmodell basierend auf einem virtuellen Zahnmodell und basierend auf Materialdaten erstellt. Dabei umfasst das virtuelle Zahnmodell zumindest virtuelle Teil-Modelle derjenigen Zähne, über die sich die Zahnschiene erstrecken soll. Derartige virtuelle Zahnmodelle können beispielsweise basierend auf Scan-Daten erstellt werden, die mittels eines Intraoral-Scanners durch Scannen von Zähnen eines Patienten erhalten werden. Die Materialdaten beschreiben wenigstens eine Eigenschaft eines Materials, aus dem die Zahnschiene hergestellt werden soll. Vereinfacht gesagt wird das virtuelle Zahnschienenrohlingmodell erstellt, indem im virtuellen Raum eine Repräsentation desjenigen Materials, aus dem die Zahnschiene hergestellt werden soll, über das virtuelle Zahnmodell gelegt wird. Das virtuelle Zahnschienenrohlingmodell ist also ein Modell oder eine Repräsentation eines Zahnschienenrohlings. Optional kann dabei auch berücksichtigt werden, mittels welcher Herstellungstechnologie die Zahnschiene hergestellt werden soll. Beispielhafte Herstellungstechnologien sind Tiefziehen, 3D-Drucken, d. h. ein generatives Herstellen der Zahnschiene, und Fräsen, d. h. ein subtraktives Herstellen der Zahnschiene. Gemäß einer zweiten Alternative wird das virtuelle Zahnschienenrohlingmodell erhalten. Das bedeutet, dass ein virtuelles Zahnschienenrohlingmodell, das an einem anderen Ort und/oder zu einer anderen Zeit und/oder mittels eines anderen Systems erstellt wurde, bereitgestellt wird. Das im Zuge des erfindungsgemäßen Verfahrens stets erhaltene virtuelle Antagonistenmodell ist dabei im Zusammenhang mit dem virtuellen Zahnmodell zu sehen. Das bedeutet, dass in einem Fall, in dem das Zahnmodell Zähne eines Unterkiefers beschreibt, das Antagonistenmodell Zähne eines zugehörigen Oberkiefers beschreibt, die den Zähnen aus dem Zahnmodell gegenüberliegen. Auch der umgekehrte Fall ist möglich. Dann beschreibt das Zahnmodell Zähne eines Oberkiefers und das Antagonistenmodell Zähne eines zugehörigen Unterkiefers. Auch das Antagonistenmodell kann beispielsweise basierend auf Scan-Daten erhalten werden, die mittels eines Intraoral-Scanners durch Scannen von Zähnen eines Patienten erhalten werden. Basierend auf dem virtuellen Zahnschienenrohlingmodell und dem virtuellen Antagonistenmodell kann nun unter Nutzung des erfindungsgemäßen Verfahrens eine Soll-Außenfläche der Zahnschiene bestimmt werden. Dabei ist eine Außenfläche der Zahnschiene diejenige Fläche, die von den Zähnen weg weist, auf denen die Zahnschiene getragen wird. Dementsprechend ist eine Innenfläche der Zahnschiene diejenige Fläche, die dazu vorgesehen ist, an denjenigen Zähnen angelegt zu werden, auf denen die Zahnschiene getragen wird. Das erfindungsgemäße Verfahren stellt somit sicher, dass die Soll-Außenfläche der Zahnschiene derart gestaltet wird, dass sie gut zum Antagonistenmodell passt. In diesem Zusammenhang werden insbesondere Bewegungseinschränkungen des Unterkiefers relativ zum Oberkiefer, die aus dem Vorhandensein der Zahnschiene resultieren, so weit wie möglich reduziert. Darüber hinaus werden in diesem Zusammenhang durch das Anpassen der Soll-Außenfläche an das Antagonistenmodell Lastspitzen reduziert, die aus einer Anlage der Außenfläche der Zahnschiene an den Antagonisten resultieren. Vereinfacht gesagt, kann durch das Berücksichtigen des Antagonistenmodells die Soll-Außenfläche derart gestaltet werden, dass die Antagonisten möglichst großflächig an der Außenfläche der Zahnschiene anliegen können. Es werden also insgesamt unerwünschte Nebeneffekte der Zahnschiene reduziert oder gänzlich vermieden und dadurch ein Tragekomfort der Zahnschiene verbessert. Aufgrund der Tatsache, dass das erfindungsgemäße Verfahren computer-implementiert ist und das Bestimmen der Soll-Außenfläche somit im virtuellen Raum erfolgt, lässt sich ferner die Herstellung der Zahnschiene vereinfachen und in ihrer Effizienz steigern. Das liegt daran, dass durch das Bestimmen der Soll-Außenfläche mittels des erfindungsgemäßen Verfahrens manuelle Nacharbeiten, die der Anpassung der Zahnschiene an die Antagonisten dienen, vollständig oder zumindest zu großen Anteilen vermieden werden können.

Die mittels des erfindungsgemäßen Verfahrens bestimmte Soll-Außenfläche der Zahnschiene kann beispielsweise in Form von Außenflächendaten, die die Soll-Außenfläche beschreiben, bereitgestellt werden. In diesem Zusammenhang können die Außenflächendaten insbesondere für ein Computer Aided Manufacturing (CAM)-System bereitgestellt werden, sodass sich in einfacher und präziser Weise eine Zahnschiene mit der bestimmten Soll-Außenfläche herstellen lässt.

Das erfindungsgemäße Verfahren wird bevorzugt im Zusammenhang mit einem Gestaltungssystem für Zahnschienen, d. h. im Zusammenhang mit einem Computer Aided Design (CAD)-System, insbesondere mit einem dentalen CAD-System, ausgeführt. Derartige Systeme sind besonders gut dafür geeignet, virtuelle Modelle zu erhalten und/oder zu erstellen und auf dieser Basis eine Soll-Außenfläche für die Zahnschiene zu bestimmen.

Das erfindungsgemäße Verfahren ist auf das Bestimmen einer Soll-Außenfläche einer Zahnschiene gerichtet. Dabei versteht es sich, dass zusätzlich auch eine Soll-Innenfläche der Zahnschiene ermittelt werden muss, sodass basierend auf der ermittelten Soll-Außenfläche und der ermittelten Soll-Innenfläche die Zahnschiene letztendlich hergestellt werden kann. Nachdem das Bestimmen der Soll-Innenfläche der Zahnschiene auf Basis des virtuellen Zahnmodells, welches zumindest virtuelle Teil-Modelle derjenigen Zähne umfasst, über die sich die Zahnschiene erstrecken soll, jedoch für sich genommen bekannt ist, wird auf das Bestimmen der Soll-Innenfläche nicht im Detail eingegangen.

Bevorzugt umfasst die Soll-Außenfläche eine Aufbissfläche. Dabei kann die Aufbissfläche auch schlicht als Bissfläche oder Kaufläche bezeichnet werden. Die Aufbissfläche beschreibt also denjenigen Abschnitt der Soll-Außenfläche der Zahnschiene, der dazu vorgesehen ist, bei zusammengebissenen Zähnen an den Antagonisten anzuliegen. Dabei kommt es nicht darauf an, ob auch auf den Antagonisten eine Zahnschiene vorgesehen ist oder nicht. Die Aufbissfläche wird als Bestandteil der Soll-Außenfläche also unter Berücksichtigung des Antagonistenmodells bestimmt. Somit ist die Aufbissfläche derart gestaltet, dass sie gut zum Antagonistenmodell passt. In diesem Zusammenhang werden insbesondere Bewegungseinschränkungen des Unterkiefers relativ zum Oberkiefer, die aus dem Vorhandensein der Zahnschiene resultieren, so weit wie möglich reduziert. Darüber hinaus werden in diesem Zusammenhang durch das Anpassen der Aufbissfläche an das Antagonistenmodell Lastspitzen reduziert, die aus einer Anlage der Aufbissfläche der Zahnschiene an den Antagonisten resultieren. Vereinfacht gesagt kann durch das Berücksichtigen des Antagonistenmodells die Aufbissfläche derart gestaltet werden, dass die Antagonisten möglichst großflächig an der Aufbissfläche der Zahnschiene anliegen können. Es werden also insgesamt unerwünschte Nebeneffekte der Zahnschiene reduziert oder gänzlich vermieden und dadurch ein Tragekomfort der Zahnschiene verbessert.

Gemäß einer Ausführungsform beschreibt das virtuelle Antagonistenmodell ferner eine Beweglichkeit der Zähne des Antagonistenmodells relativ zu den Zähnen des virtuellen Zahnmodells. Das bedeutet, dass das Antagonistenmodell nicht nur Daten umfasst, die eine Geometrie der Antagonisten beschreiben, sondern auch die Beweglichkeit der Zähne des Antagonistenmodells relativ zu den Zähnen des virtuellen Zahnmodells beschreibende Daten.

Diese Beweglichkeit kann beispielsweise durch eine Kurve beschrieben werden, entlang der die Zähne des Antagonistenmodells relativ zu den Zähnen des virtuellen Zahnmodells bewegt werden. Alternativ kann die Beweglichkeit durch eine Gruppe an Punkten beschrieben werden, die ausgewählte Relativpositionen des Antagonistenmodells relativ zum virtuellen Zahnmodell beschreiben. Gemäß einer weiteren Alternative kann die Beweglichkeit als Ergebnis einer Bewegungssimulation erhalten werden, wobei im Zuge der Bewegungssimulation eine Relativbewegung zwischen Oberkiefer und Unterkiefer nachgestellt wird. Es versteht sich, dass es in diesem Zusammenhang lediglich auf die Relativbewegung ankommt. Auf diese Weise kann die Beweglichkeit unabhängig davon beschrieben werden, ob die Zahnschiene für einen Unterkiefer vorgesehen ist oder für einen Oberkiefer. Die Berücksichtigung der Beweglichkeit führt dazu, dass unerwünschte Nebeneffekte, die aus dem Tragen der Zahnschiene resultieren, weiter reduziert werden. Insbesondere werden auf diese Weise Einschränkungen auf die Beweglichkeit des Unterkiefers und des Oberkiefers relativ zueinander verringert. In einem Spezialfall kann die Zahnschiene dazu vorgesehen sein, bei einem Patienten die Beweglichkeit des Unterkiefers relativ zum Oberkiefer zu beeinflussen. In einem solchen Fall dient die Berücksichtigung der Beweglichkeit beim Bestimmen der Soll-Außenfläche ferner dazu, dass diese Art der Beeinflussung der Beweglichkeit zuverlässig gewährleistet werden kann.

Das Verfahren kann ferner das Erhalten von Patientendaten umfassen, welche die Beweglichkeit der Zähne des Antagonistenmodells relativ zu den Zähnen des virtuellen Zahnmodells beschreiben. Mit anderen Worten kann die Beweglichkeit der Zähne des Antagonistenmodells relativ zu den Zähnen des virtuellen Zahnmodells patientenindividuell beschrieben sein. Wenn eine solche Beweglichkeit beim Bestimmen der Soll-Außenfläche berücksichtigt wird, führt das zu einer Zahnschiene mit besonders guter Passform, die besonders wenige unerwünschte Nebeneffekte bewirkt, welche sich auf die Beweglichkeit des Unterkiefers relativ zum Oberkiefer auswirken.

In einem Beispiel beschreiben die Materialdaten ein Tiefziehmaterial. Das Erzeugen eines virtuellen Zahnschienenrohlingmodells umfasst ein Ausführen einer Tiefziehsimulation, bei der ein Tiefziehen des Tiefziehmaterials über das Zahnmodell nachgestellt wird. Es wird also ein virtuelles Zahnschienenrohlingmodell erzeugt, das eine tiefgezogene Zahnschiene beschreibt. Dabei ist das Tiefziehen von Zahnschienen für sich genommen bekannt und bewährt. Insbesondere ist das Tiefziehen von Zahnschienen hinsichtlich der Innenfläche der Zahnschiene vorteilhaft, da auf diese Weise eine präzise Anpassung der Zahnschiene auf diejenigen Zähne, auf denen die Zahnschiene getragen werden soll, leicht möglich ist. Beim Tiefziehen gemäß bekannter Verfahren ergibt sich jedoch die Außenfläche der Zahnschiene aus dem Prozess, ohne dass die Außenfläche gesondert gestaltet wird. Für den Fall, dass die Tiefziehsimulation im Zusammenhang mit dem erfindungsgemäßen Verfahren ausgeführt wird, ist das jedoch nicht so. Vielmehr wird dann, wie bereits erläutert, die Soll-Außenfläche unter anderem unter Berücksichtigung des virtuellen Antagonistenmodells erstellt. Mit anderen Worten wird auch eine gute Passform hinsichtlich der Antagonisten erreicht. Zusammengenommen bewirken also die Tiefziehsimulation und das Bestimmen der Soll-Außenfläche, dass die Zahnschiene sowohl hinsichtlich ihrer Innenfläche als auch hinsichtlich ihrer Außenfläche präzise an den Patienten angepasst werden kann und somit insgesamt einen guten Tragekomfort aufweist, d. h. wenig unerwünschte Nebeneffekte hat.

Dabei kann eine Eigenschaft des Materials, aus dem die Zahnschiene hergestellt werden soll, einen Materialhersteller, einen Materialtyp, eine Materialdicke und/oder eine mechanische Materialeigenschaft beschreiben. Die Berücksichtigung dieser Eigenschaften bewirkt, dass das virtuelle Zahnschienenrohlingmodell mit hoher Präzision erstellt werden kann. Dies wird insbesondere anhand eines Beispiels deutlich, bei dem die Zahnschiene mittels eines Tiefziehverfahrens hergestellt werden soll. In diesem Zusammenhang ist also das Material ein Tiefziehmaterial, das beispielsweise in Form einer Folie bereitgestellt wird. Diese Folie ist durch einen zugehörigen Materialhersteller, einen Materialtyp, eine Materialdicke und/oder eine mechanische Materialeigenschaft, beispielsweise einen Elastizitätsmodul beschrieben. Basierend hierauf kann, zum Beispiel mittels einer Tiefziehsimulation, das virtuelle Zahnschienenrohlingmodell präzise und zuverlässig erzeugt werden. Nachdem das virtuelle Zahnschienenrohlingmodell beim Bestimmen der Soll-Außenfläche berücksichtigt wird, ergibt sich ferner eine zuverlässig und präzise bestimmte Soll-Außenfläche.

Das Verfahren kann ferner ein virtuelles Positionieren von einer oder mehreren Durchgangsöffnungen in der Zahnschiene umfassen. Das erfolgt basierend auf dem virtuellen Zahnmodell. Alternativ oder zusätzlich kann das Verfahren ein virtuelles Positionieren wenigstens eines Abschnitts eines Saumes der Zahnschiene umfassen. Auch das erfolgt basierend auf dem virtuellen Zahnmodell. Die Durchgangsöffnungen können dabei für Speichel und/oder Nahrungspartikel sein. Die Zahnschiene hat also mehrere Löcher oder Öffnungen, die so dimensioniert sind, dass ein Speichelaustausch sowie ein Transfer von Nahrungspartikeln von einer Seite auf eine andere Seite der Zahnschiene möglich sind. Derartige Zahnschienen haben mehrere zahnmedizinische Vorteile. Was die Nahrungspartikel betrifft, so ist es mit derartigen Zahnschienen möglich, die Nahrungspartikel durch die Durchgangsöffnungen aus dem Bereich der Zähne abzuführen. Mit anderen Worten wird durch die Durchgangsöffnungen verhindert, dass Nahrungspartikel mittels der Zahnschiene im Bereich der Zähne gehalten werden. Das reduziert die Gefahr für Karies und Gingivitis. Auch der Austausch von Speichel zwischen derjenigen Seite der Zahnschiene, die an den Zähnen anliegt, und derjenigen Seite der Zahnschiene, die von den Zähnen weg weist, ist vorteilhaft. Ein derartiger Austausch von Speichel reduziert die Bildung von Zahnbelägen sowie das Risiko von Karies und Gingivitis. Auch in Fällen, in denen ein Intraoralsensor zur Detektion von Biomarkern im Speichel verwendet wird, ist eine Zahnschiene mit Durchgangsöffnungen vorteilhaft, da so für ausreichenden Speichelfluss im Bereich des Sensors gesorgt werden kann, sodass dieser zuverlässige Messergebnisse liefern kann. Unter einem Saum werden in diesem Zusammenhang diejenigen Endkanten oder Endflächen der Zahnschiene verstanden, die üblicherweise in einem Grenzbereich zwischen Zähnen und Zahnfleisch angeordnet sind, sofern die Zahnschiene getragen wird. Der Saum kann mittels Schneiden oder Spanen hergestellt werden. Alternativ kann der Saum durch Vorsehen einer Sollbruchstelle zum Trennen der Zahnschiene von überschüssigem Material gestaltet sein. Mittels einer derartigen Sollbruchstelle können also nach dem Herstellen der Zahnschiene diejenigen Abschnitte des Materials, die die Zahnschiene bilden, von den übrigen Abschnitten des Materials getrennt werden. Dieser Trennvorgang ist aufgrund der Sollbruchstelle besonders einfach und zuverlässig. Beispielsweise ist die Sollbruchstelle durch eine Perforation gebildet. Darüber hinaus kann die Sollbruchstelle patientenindividuell angeordnet und/oder gestaltet werden. Somit ergibt sich ohne weitere Nachbearbeitungsschritte ein hoher Tragekomfort der Zahnschiene.

In einem Ausführungsbeispiel wird beim Bestimmen der Soll-Außenfläche der Zahnschiene basierend auf dem virtuellen Zahnschienenrohlingmodell und dem virtuellen Antagonistenmodell ferner eine vorgegebene Mindestdicke der Zahnschiene berücksichtigt. Die Mindestdicke kann abhängig vom Material sein, aus dem die Zahnschiene hergestellt werden soll. Durch das Berücksichtigen der Mindestdicke wird sichergestellt, dass die Zahnschiene die Mindestdicke nicht unterschreitet und somit ein Mindestmaß an mechanischer Stabilität aufweist. Auf diese Weise kann gewährleistet werden, dass die Zahnschiene über einen vorgegebenen Zeitraum funktionstüchtig ist. Die Dicken in den übrigen Abschnitten der Zahnschiene werden ausgehend von der vorgegeben Mindestdicke bestimmt. Dabei ist es häufig, dass die Mindestdicke im Bereich des hintersten Backenzahns vorliegt.

Die Aufgabe wird ferner durch ein Verfahren zum Herstellen einer Zahnschiene gelöst. Das Verfahren umfasst
- Erhalten von Außenflächendaten, die zumindest einen Abschnitt einer Soll-Außenfläche der Zahnschiene beschreiben, insbesondere wobei die Außenflächendaten mittels eines erfindungsgemäßen Verfahrens zum Bestimmen einer Soll-Außenfläche einer Zahnschiene bestimmt sind, und
- Angleichen einer Ist-Außenfläche der Zahnschiene an die Soll-Außenfläche durch spanendes Bearbeiten.

Wie bisher ist dabei eine Außenfläche der Zahnschiene diejenige Fläche, die von den Zähnen weg weist, auf denen die Zahnschiene getragen wird. Dementsprechend ist eine Innenfläche der Zahnschiene diejenige Fläche, die dazu vorgesehen ist, an denjenigen Zähnen angelegt zu werden, auf denen die Zahnschiene getragen wird. Durch Nutzung des erfindungsgemäßen Verfahrens zum Herstellen einer Zahnschiene kann somit basierend auf den Außenflächendaten die Außenfläche der Zahnschiene derart gestaltet werden, dass sie gut zu den Antagonisten passt. In diesem Zusammenhang werden insbesondere Bewegungseinschränkungen des Unterkiefers relativ zum Oberkiefer, die aus dem Vorhandensein der Zahnschiene resultieren, so weit wie möglich reduziert. Darüber hinaus werden in diesem Zusammenhang Lastspitzen reduziert, die aus einer Anlage der Außenfläche der Zahnschiene an den Antagonisten resultieren. Vereinfacht gesagt kann basierend auf den Außenflächendaten die Außenfläche derart gestaltet werden, dass die Antagonisten möglichst großflächig an der Außenfläche der Zahnschiene anliegen können. Es werden also insgesamt unerwünschte Nebeneffekte der Zahnschiene reduziert oder gänzlich vermieden und dadurch ein Tragekomfort der Zahnschiene verbessert. Aufgrund der Tatsache, dass das Angleichen der Ist-Außenfläche an die Soll-Außenfläche basierend auf Außenflächendaten spanend erfolgt, lässt sich ferner die Herstellung der Zahnschiene vereinfachen und in ihrer Effizienz steigern, insbesondere indem das Verfahren automatisiert abläuft. Manuelle Arbeiten, insbesondere manuelle Nacharbeiten, die der Anpassung der Zahnschiene an die Antagonisten dienen, können somit vollständig oder zumindest zu großen Anteilen vermieden werden.

Gemäß einer bevorzugten Ausführungsform sind die Außenflächendaten mittels eines erfindungsgemäßen Verfahrens zum Bestimmen einer Soll-Außenfläche einer Zahnschiene bestimmt. Die Soll-Außenfläche ist also mittels des bereits erläuterten, erfindungsgemäßen Verfahrens zum Bestimmen einer Soll-Außenfläche bestimmt. Die Soll-Außenfläche wird durch die Außenflächendaten präzise und zuverlässig beschrieben.

Es versteht sich, dass das erfindungsgemäße Verfahren zum Herstellen einer Zahnschiene prinzipiell unabhängig von der Herstellungstechnologie für die Zahnschiene ist. Beispielsweise kann die Zahnschiene mittels eines Tiefziehverfahrens, mittels eines additiven Herstellungsverfahrens oder mittels eines subtraktiven Herstellungsverfahrens hergestellt werden, bevor die Ist-Außenfläche der Zahnschiene durch ein spanendes Bearbeiten an die Soll-Außenfläche angeglichen wird. Besonders vorteilhaft ist in diesem Zusammenhang jedoch, zunächst ein Tiefziehverfahren anzuwenden, das bewirkt, dass eine Innenfläche der Zahnschiene präzise gestaltet werden kann, sodass diese gut zu denjenigen Zähnen passt, auf denen die Zahnschiene getragen werden soll. Anschließend wird dann mittels spanender Bearbeitung die Ist-Außenfläche an die Soll-Außenfläche angepasst. Somit lässt sich die Außenfläche an die zugeordneten Antagonisten anpassen. Insgesamt werden also die Vorteile des Tiefziehens für die Innenfläche und die Vorteile der spanenden Bearbeitung für die Außenfläche kombiniert. Ähnliches gilt auch für Zahnschienen, die mittels 3D-Druck hergestellt werden.

Bevorzugt umfasst die Soll-Außenfläche eine Aufbissfläche. Dabei kann die Aufbissfläche auch schlicht als Bissfläche oder Kaufläche bezeichnet werden. Die Aufbissfläche beschreibt also denjenigen Abschnitt der Soll-Außenfläche der Zahnschiene, der dazu vorgesehen ist, bei zusammengebissenen Zähnen an den Antagonisten anzuliegen. Dabei kommt es nicht darauf an, ob auch auf den Antagonisten eine Zahnschiene vorgesehen ist oder nicht. Die Aufbissfläche wird als Bestandteil der Soll-Außenfläche, also unter Berücksichtigung des Antagonistenmodells bestimmt. Somit ist die Aufbissfläche derart gestaltet, dass sie gut zum Antagonistenmodell passt. In diesem Zusammenhang werden insbesondere Bewegungseinschränkungen des Unterkiefers relativ zum Oberkiefer, die aus dem Vorhandensein der Zahnschiene resultieren, so weit wie möglich reduziert. Darüber hinaus werden in diesem Zusammenhang durch das Anpassen der Aufbissfläche an das Antagonistenmodell Lastspitzen reduziert, die aus einer Anlage der Aufbissfläche der Zahnschiene an den Antagonisten resultieren. Vereinfacht gesagt, kann durch das Berücksichtigen des Antagonistenmodells die Aufbissfläche derart gestaltet werden, dass die Antagonisten möglichst großflächig an der Aufbissfläche der Zahnschiene anliegen können. Es werden also insgesamt unerwünschte Nebeneffekte der Zahnschiene reduziert oder gänzlich vermieden und dadurch ein Tragekomfort der Zahnschiene verbessert.

Gemäß einem Beispiel erfolgt das Angleichen der Ist-Außenfläche an die Soll-Außenfläche, während die Zahnschiene auf einem Tiefziehwerkzeug gelagert ist oder während die Zahnschiene auf einer vom Tiefziehwerkzeug separaten Vorrichtung gelagert ist. Das Tiefziehwerkzeug oder die separate Vorrichtung kann dabei ganz oder teilweise als physisches Zahnmodell ausgestaltet sein. Bevorzugt ist dieses Tiefziehwerkzeug oder die separate Vorrichtung zumindest abschnittsweise mittels eines 3D-Druck-Verfahrens hergestellt. In beiden Varianten lässt sich folglich die Ist-Außenfläche einfach und präzise an die Soll-Außenfläche angleichen. In einem Fall, in dem das Angleichen erfolgt, während die Zahnschiene auf dem Tiefziehwerkzeug gelagert ist, ist das Tiefziehwerkzeug gleichzeitig eine Aufspannvorrichtung oder Lagervorrichtung für das Angleichen der Ist-Außenfläche an die Soll-Außenfläche. In diesem Zusammenhang können zudem Arbeitsschritte entfallen, die ein Umlagern oder Umsetzen der Zahnschiene betreffen. Das erhöht weiter die Effizienz.

In einem Fall, in dem das Angleichen der Ist-Außenfläche an die Soll-Außenfläche erfolgt, während die Zahnschiene auf einem Tiefziehwerkzeug gelagert ist, d.h. in einem Fall, in dem das Tiefziehwerkzeug eine Aufspannvorrichtung oder Lagervorrichtung für das Angleichen der Ist-Außenfläche an die Soll-Außenfläche ist, ist es zudem möglich, dass eine Bauplattform, auf der das physische Zahnmodell mittels 3D-Druck hergestellt wird, ebenfalls ein Bestandteil des Tiefziehwerkzeugs und somit auch der Aufspannvorrichtung oder Lagervorrichtung ist. In diesem Fall wird also das physische Zahnmodell mittels 3D-Druck auf der Bauplattform hergestellt. Sodann wird die Bauplattform zusammen mit dem physischen Zahnmodell als Tiefziehwerkzeug verwendet. Anschließend wird die Bauplattform mit dem physischen Zahnmodell als Aufspannvorrichtung oder Lagervorrichtung für das Angleichen der Ist-Außenfläche an die Soll-Außenfläche verwendet. Die Bauplattform wird somit durchgängig für mehrere, insbesondere alle, Arbeitsschritte verwendet, die zum Herstellen der Zahnschiene nötig sind. Dabei ist die Bauplattform dazu ausgebildet, mit der nötigen Präzision in den für die jeweiligen Arbeitsschritte verwendeten Maschinen und Anlagen positioniert und orientiert zu werden. Optional gilt das auch für einen Reinigungsschritt, der in einer Reinigungsanlage ausgeführt wird, und/oder einen Nachvergütungsschritt, der in einer Nachvergütungsanlage ausgeführt wird. Eine derartige Bauplattform ist somit universell für mehrere oder alle Maschinen und Anlagen verwendbar, die dem Herstellen der Zahnschiene dienen. Folglich muss die Zahnschiene während mehrerer, insbesondere während aller Arbeitsschritte nicht umgelagert oder umgesetzt werden. Das erhöht weiter die Effizienz bei der Herstellung der Zahnschiene. Ferner wird eine Qualität der Zahnschiene dadurch positiv beeinflusst, was nicht zuletzt daran liegt, dass Positionierungsfehler, die beim Umsetzen oder Umlagern auftreten können, zuverlässig vermieden werden können.

Das Verfahren kann ferner ein Einbringen von einer oder mehreren Durchgangsöffnungen durch spanendes Bearbeiten umfassen. Alternativ oder zusätzlich kann das Verfahren ein Erstellen wenigstens eines Abschnitts eines Saumes der Zahnschiene durch spanendes Bearbeiten umfassen. Weiter alternativ oder zusätzlich kann das Verfahren ein zumindest abschnittsweises Polieren der Zahnschiene umfassen. Wie zuvor erwähnt, können die Durchgangsöffnungen für Speichel und/oder Nahrungspartikel sein. Die Zahnschiene hat also mehrere Löcher oder Öffnungen, die so dimensioniert sind, dass ein Speichelaustausch sowie ein Transfer von Nahrungspartikeln von einer Seite auf eine andere Seite der Zahnschiene möglich sind. Derartige Zahnschienen haben mehrere zahnmedizinische Vorteile. Was die Nahrungspartikel betrifft, so ist es mit derartigen Zahnschienen möglich, die Nahrungspartikel durch die Durchgangsöffnungen aus dem Bereich der Zähne abzuführen. Mit anderen Worten wird durch die Durchgangsöffnungen verhindert, dass Nahrungspartikel mittels der Zahnschiene im Bereich der Zähne gehalten werden. Das reduziert die Gefahr für Karies und Gingivitis. Auch der Austausch von Speichel zwischen derjenigen Seite der Zahnschiene, die an den Zähnen anliegt, und derjenigen Seite der Zahnschiene, die von den Zähnen weg weist, ist vorteilhaft. Ein derartiger Austausch von Speichel reduziert die Bildung von Zahnbelägen sowie das Risiko von Karies und Gingivitis. Auch in Fällen, in denen ein Intraoralsensor zur Detektion von Biomarkern im Speichel verwendet wird, ist eine Zahnschiene mit Durchgangsöffnungen vorteilhaft, da so für ausreichenden Speichelfluss im Bereich des Sensors gesorgt werden kann, sodass dieser zuverlässige Messergebnisse liefern kann. Unter einem Saum werden in diesem Zusammenhang diejenigen Endkanten oder Endflächen der Zahnschiene verstanden, die üblicherweise in einem Grenzbereich zwischen Zähnen und Zahnfleisch angeordnet sind, sofern die Zahnschiene getragen wird. Wenn der Saum mittels eines spanenden Verfahrens hergestellt wird, kann er direkt vor oder nach dem Angleichen der Ist-Außenfläche an die Soll-Außenfläche hergestellt werden, was einfach und effizient ist. Alternativ kann der Saum durch Vorsehen einer Sollbruchstelle zum Trennen der Zahnschiene von überschüssigem Material gestaltet sein. Auch diese Sollbruchstelle kann spanend hergestellt werden. Mittels einer derartigen Sollbruchstelle können also nach dem Herstellen der Zahnschiene diejenigen Abschnitte des Materials, die die Zahnschiene bilden, von den übrigen Abschnitten des Materials getrennt werden. Dieser Trennvorgang ist aufgrund der Sollbruchstelle besonders einfach und zuverlässig. Beispielsweise ist die Sollbruchstelle durch eine Perforation gebildet. Darüber hinaus kann die Sollbruchstelle patientenindividuell angeordnet und/oder gestaltet werden. Somit ergibt sich ohne weitere Nachbearbeitungsschritte ein hoher Tragekomfort der Zahnschiene. Auch das Polieren kann direkt vor oder nach dem Angleichen der Ist-Außenfläche an die Soll-Außenfläche erfolgen. Somit wird die Effizienz beim Herstellen der Zahnschiene weiter gesteigert.

Außerdem wird die Aufgabe durch eine Zahnschiene gelöst, die mittels eines erfindungsgemäßen Verfahrens zum Herstellen einer Zahnschiene erhalten ist. Durch Nutzung des erfindungsgemäßen Verfahrens zum Herstellen einer Zahnschiene kann somit basierend auf den Außenflächendaten die Außenfläche der Zahnschiene derart gestaltet werden, dass sie gut zu den Antagonisten passt. In diesem Zusammenhang werden insbesondere Bewegungseinschränkungen des Unterkiefers relativ zum Oberkiefer, die aus dem Vorhandensein der Zahnschiene resultieren, so weit wie möglich reduziert. Darüber hinaus werden in diesem Zusammenhang Lastspitzen reduziert, die aus einer Anlage der Außenfläche der Zahnschiene an den Antagonisten resultieren. Vereinfacht gesagt kann basierend auf den Außenflächendaten die Außenfläche derart gestaltet werden, dass die Antagonisten möglichst großflächig an der Außenfläche der Zahnschiene anliegen können. Es werden also insgesamt unerwünschte Nebeneffekte der Zahnschiene reduziert oder gänzlich vermieden und dadurch ein Tragekomfort der Zahnschiene verbessert. Aufgrund der Tatsache, dass das Angleichen der Ist-Außenfläche an die Soll-Außenfläche basierend auf Außenflächendaten spanend erfolgt, lässt sich ferner die Herstellung der Zahnschiene vereinfachen und in ihrer Effizienz steigern, insbesondere indem das Verfahren automatisiert abläuft. Manuelle Arbeiten, insbesondere manuelle Nacharbeiten, die der Anpassung der Zahnschiene an die Antagonisten dienen, können somit vollständig oder zumindest zu großen Anteilen vermieden werden.

Zudem wird die Aufgabe durch eine Vorrichtung zur Datenverarbeitung gelöst, die Mittel zur Ausführung des erfindungsgemäßen Verfahrens zum Bestimmen einer Soll-Außenfläche einer Zahnschiene umfasst. Basierend auf dem virtuellen Zahnschienenrohlingmodell und dem virtuellen Antagonistenmodell, die im erfindungsgemäßen Verfahren zum Bestimmen einer Soll-Außenfläche einer Zahnschiene berücksichtigt werden, kann unter Nutzung der Vorrichtung zur Datenverarbeitung eine Soll-Außenfläche der Zahnschiene bestimmt werden. Dabei ist eine Außenfläche der Zahnschiene diejenige Fläche, die von den Zähnen weg weist, auf denen die Zahnschiene getragen wird. Dementsprechend ist eine Innenfläche der Zahnschiene diejenige Fläche, die dazu vorgesehen ist, an denjenigen Zähnen angelegt zu werden, auf denen die Zahnschiene getragen wird. Dadurch wird sichergestellt, dass die Soll-Außenfläche der Zahnschiene derart gestaltet wird, dass sie gut zum Antagonistenmodell passt. In diesem Zusammenhang werden insbesondere Bewegungseinschränkungen des Unterkiefers relativ zum Oberkiefer, die aus dem Vorhandensein der Zahnschiene resultieren, so weit wie möglich reduziert. Darüber hinaus werden in diesem Zusammenhang durch das Anpassen der Soll-Außenfläche an das Antagonistenmodell Lastspitzen reduziert, die aus einer Anlage der Außenfläche der Zahnschiene an den Antagonisten resultieren. Vereinfacht gesagt kann durch das Berücksichtigen des Antagonistenmodells die Soll-Außenfläche derart gestaltet werden, dass die Antagonisten möglichst großflächig an der Außenfläche der Zahnschiene anliegen können. Es werden also insgesamt unerwünschte Nebeneffekte der Zahnschiene reduziert oder gänzlich vermieden und dadurch ein Tragekomfort der Zahnschiene verbessert.

Zusätzlich wird die Aufgabe durch ein Computerprogramm gelöst, das Befehle umfasst, die bei der Ausführung des Computerprogramms durch einen Computer diesen veranlassen, das erfindungsgemäße Verfahren zum Bestimmen einer Soll-Außenfläche einer Zahnschiene auszuführen. Basierend auf dem virtuellen Zahnschienenrohlingmodell und dem virtuellen Antagonistenmodell, die im erfindungsgemäßen Verfahren zum Bestimmen einer Soll-Außenfläche einer Zahnschiene berücksichtigt werden, kann unter Nutzung des Computerprogramms eine Soll-Außenfläche der Zahnschiene bestimmt werden. Dabei ist eine Außenfläche der Zahnschiene diejenige Fläche, die von den Zähnen weg weist, auf denen die Zahnschiene getragen wird. Dementsprechend ist eine Innenfläche der Zahnschiene diejenige Fläche, die dazu vorgesehen ist, an denjenigen Zähnen angelegt zu werden, auf denen die Zahnschiene getragen wird. Dadurch wird sichergestellt, dass die Soll-Außenfläche der Zahnschiene derart gestaltet wird, dass sie gut zum Antagonistenmodell passt. In diesem Zusammenhang werden insbesondere Bewegungseinschränkungen des Unterkiefers relativ zum Oberkiefer, die aus dem Vorhandensein der Zahnschiene resultieren, so weit wie möglich reduziert. Darüber hinaus werden in diesem Zusammenhang durch das Anpassen der Soll-Außenfläche an das Antagonistenmodell Lastspitzen reduziert, die aus einer Anlage der Außenfläche der Zahnschiene an den Antagonisten resultieren. Vereinfacht gesagt kann durch das Berücksichtigen des Antagonistenmodells die Soll-Außenfläche derart gestaltet werden, dass die Antagonisten möglichst großflächig an der Außenfläche der Zahnschiene anliegen können. Es werden also insgesamt unerwünschte Nebeneffekte der Zahnschiene reduziert oder gänzlich vermieden und dadurch ein Tragekomfort der Zahnschiene verbessert.

Ebenfalls wird die Aufgabe durch ein computerlesbares Medium gelöst, das Befehle umfasst, die bei der Ausführung durch einen Computer diesen veranlassen, das erfindungsgemäße Verfahren zum Bestimmen einer Soll-Außenfläche einer Zahnschiene auszuführen. Basierend auf dem virtuellen Zahnschienenrohlingmodell und dem virtuellen Antagonistenmodell, die im erfindungsgemäßen Verfahren zum Bestimmen einer Soll-Außenfläche einer Zahnschiene berücksichtigt werden, kann unter Nutzung des computerlesbaren Mediums eine Soll-Außenfläche der Zahnschiene bestimmt werden. Dabei ist eine Außenfläche der Zahnschiene diejenige Fläche, die von den Zähnen weg weist, auf denen die Zahnschiene getragen wird. Dementsprechend ist eine Innenfläche der Zahnschiene diejenige Fläche, die dazu vorgesehen ist, an denjenigen Zähnen angelegt zu werden, auf denen die Zahnschiene getragen wird. Dadurch wird sichergestellt, dass die Soll-Außenfläche der Zahnschiene derart gestaltet wird, dass sie gut zum Antagonistenmodell passt. In diesem Zusammenhang werden insbesondere Bewegungseinschränkungen des Unterkiefers relativ zum Oberkiefer, die aus dem Vorhandensein der Zahnschiene resultieren, so weit wie möglich reduziert. Darüber hinaus werden in diesem Zusammenhang durch das Anpassen der Soll-Außenfläche an das Antagonistenmodell Lastspitzen reduziert, die aus einer Anlage der Außenfläche der Zahnschiene an den Antagonisten resultieren. Vereinfacht gesagt kann durch das Berücksichtigen des Antagonistenmodells die Soll-Außenfläche derart gestaltet werden, dass die Antagonisten möglichst großflächig an der Außenfläche der Zahnschiene anliegen können. Es werden also insgesamt unerwünschte Nebeneffekte der Zahnschiene reduziert oder gänzlich vermieden und dadurch ein Tragekomfort der Zahnschiene verbessert.

Es versteht sich, dass Merkmale, Effekte und/oder Vorteile, die im Zusammenhang mit einem aus erfindungsgemäßem Verfahren zum Bestimmen einer Soll-Außenfläche, erfindungsgemäßem Verfahren zum Herstellen einer Zahnschiene, erfindungsgemäßer Zahnschiene, erfindungsgemäßer Vorrichtung zur Datenverarbeitung, erfindungsgemäßem Computerprogramm und erfindungsgemäßem computerlesbaren Medium erwähnt wurden, auch für die jeweils anderen aus erfindungsgemäßem Verfahren zum Bestimmen einer Soll-Außenfläche, erfindungsgemäßem Verfahren zum Herstellen einer Zahnschiene, erfindungsgemäßer Zahnschiene, erfindungsgemäßer Vorrichtung zur Datenverarbeitung, erfindungsgemäßem Computerprogramm und erfindungsgemäßem computerlesbaren Medium gelten.

Die Erfindung wird nachstehend anhand verschiedener Ausführungsbeispiele erläutert, die in den beigefügten Zeichnungen gezeigt sind. Es zeigen:
- Figur 1: eine erfindungsgemäße Vorrichtung zur Datenverarbeitung mit einem erfindungsgemäßen computerlesbaren Medium und einem erfindungsgemäßen Computerprogramm, wobei die Vorrichtung zur Datenverarbeitung dazu ausgebildet ist, ein erfindungsgemäßes Verfahren zum Bestimmen einer Soll-Außenfläche einer Zahnschiene auszuführen und wobei die Vorrichtung zur Datenverarbeitung mit einer Herstellungsvorrichtung gekoppelt ist, sodass sich eine erfindungsgemäße Zahnschiene unter Nutzung eines erfindungsgemäßen Verfahrens zur Herstellung einer Zahnschiene herstellen lässt,
- Figur 2: schematisch ein virtuelles Zahnmodell, ein virtuelles Antagonistenmodell und ein virtuelles Zahnschienenrohlingmodell, wobei die Soll-Außenfläche der Zahnschiene noch nicht bestimmt ist,
- Figur 3: schematisch das virtuelle Zahnmodell, das virtuelle Antagonistenmodell und das virtuelle Zahnschienenrohlingmodell aus Figur 2, wobei die Soll-Außenfläche bestimmt wurde,
- Figur 4: schematisch das virtuelle Zahnmodell, das virtuelle Antagonistenmodell und das virtuelle Zahnschienenrohlingmodell aus Figur 3, wobei ein geschlossener Zustand des aus Zahnmodell und Antagonistenmodell gebildeten Gebissmodells gezeigt ist,
- Figur 5: einen Zwischenzustand einer erfindungsgemäßen Zahnschiene direkt nach dem Ausführen eines Tiefziehverfahrens,
- Figur 6: in einer Detailansicht einen weiteren Zwischenzustand der erfindungsgemäßen Zahnschiene, wobei zumindest ein Teil der Ist-Außenfläche der Zahnschiene an die Soll-Außenfläche durch spanendes Bearbeiten angeglichen ist, und
- Figur 7: die erfindungsgemäße Zahnschiene in einer separaten Darstellung.

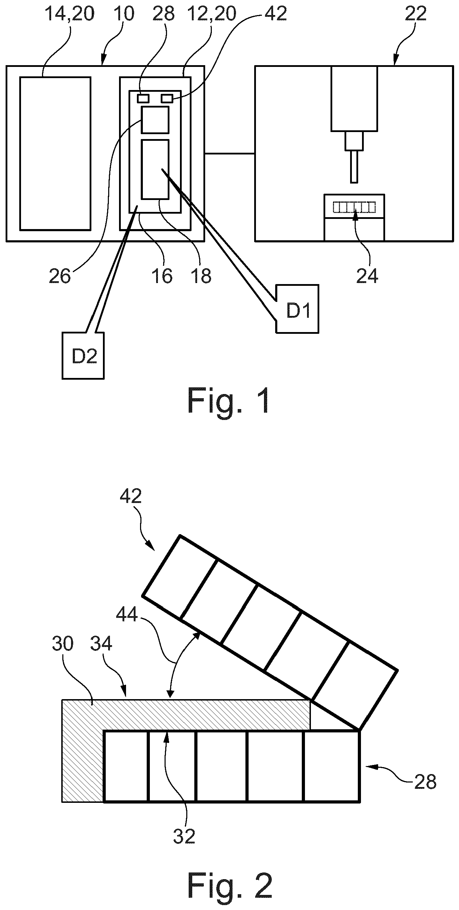

Figur 1 zeigt eine Vorrichtung 10 zur Datenverarbeitung.

Diese umfasst eine Speichereinheit 12 und eine Recheneinheit 14.

Dabei weist die Speichereinheit 12 ein computerlesbares Medium 16 auf.

Auf dem computerlesbaren Medium 16, d. h. auch auf der Speichereinheit 12, ist ein Computerprogramm 18 gespeichert.

Das Computerprogramm 18 und somit auch das computerlesbare Medium 16 umfassen Befehle, die bei der Ausführung des Computerprogramms 18 durch die Recheneinheit 14 oder, allgemeiner gesprochen, durch einen Computer, die Recheneinheit 14 oder den Computer dazu veranlassen, ein Verfahren zum Bestimmen einer Soll-Außenfläche einer Zahnschiene auszuführen.

Folglich stellen die Speichereinheit 12 und die Recheneinheit 14 Mittel 20 zum Ausführen des Verfahrens zum Bestimmen einer Soll-Außenfläche einer Zahnschiene dar.

Im Beispiel der Figur 1 ist die Vorrichtung 10 zur Datenverarbeitung ferner kommunikationstechnisch mit einer Herstellungsvorrichtung 22 gekoppelt. Dabei ist die Herstellungsvorrichtung 22 dazu ausgebildet, eine Zahnschiene 24 spanend zu bearbeiten, sodass eine Ist-Außenfläche der Zahnschiene 24 an eine Soll-Außenfläche angepasst werden kann, welche mittels des Verfahrens zum Bestimmen der Soll-Außenfläche der Zahnschiene bestimmt wurde.

Aus diesem Grund umfasst die Speichereinheit 12 im dargestellten Ausführungsbeispiel zusätzlich ein Computerprogramm 26 zum Ansteuern der Herstellungsvorrichtung 22. Mit anderen Worten umfasst das Computerprogramm 26 Befehle, die bei der Ausführung des Computerprogramms 26 durch die Recheneinheit 14 oder, allgemeiner gesprochen, durch einen Computer, die Recheneinheit 14 oder den Computer dazu veranlassen, die Herstellungsvorrichtung 22 zu steuern.

Im Beispiel der Figur 1 ist die Herstellungsvorrichtung 22 dazu ausgebildet, die Zahnschiene 24 spanend zu bearbeiten. Konkreter gesagt, handelt es sich bei der Herstellungsvorrichtung 22 um eine Fräsmaschine.

Im Folgenden wird ein Verfahren zum Herstellen einer Zahnschiene 24 anhand der Figuren 2 bis 7 im Detail erläutert.

Das Verfahren zum Herstellen einer Zahnschiene 24 umfasst zunächst das Bestimmen der Soll-Außenfläche der Zahnschiene 24. Hierfür wird ein Verfahren zum Bestimmen der Soll-Außenfläche der Zahnschiene 24 verwendet, dessen Ergebnis in Form von Außenflächendaten D1 bereitgestellt wird, die die bestimmte Soll-Außenfläche beschreiben.

Im Zuge des computer-implementierten Verfahrens zum Bestimmen einer Soll-Außenfläche der Zahnschiene 24 wird zunächst ein virtuelles Zahnmodell 28 erhalten.

Das virtuelle Zahnmodell 28 umfasst virtuelle Modelle derjenigen Zähne, über die sich die Zahnschiene 24 erstrecken soll.

In der dargestellten Ausführungsform soll sich die Zahnschiene über den gesamten Unterkiefer erstrecken. Somit umfasst das virtuelle Zahnmodell 28 in der dargestellten Ausführungsform Modelle aller Zähne des Unterkiefers.

Das virtuelle Zahnmodell 28 ist dabei basierend auf patientenindividuellen Scan-Daten erstellt, die mittels eines Intraoralscans an demjenigen Patienten generiert wurden, für den die Zahnschiene 24 hergestellt werden soll.

Darüber hinaus werden Materialdaten D2 erhalten, die wenigstens eine Eigenschaft eines Materials beschreiben, aus dem die Zahnschiene 24 hergestellt werden soll.

Im dargestellten Ausführungsbeispiel umfasst das Herstellen der Zahnschiene 24 ein Tiefziehen eines folienförmigen Tiefziehmaterials. Dementsprechend beschreiben vorliegend die Materialdaten D2 ein Tiefziehmaterial, das eine Folie ist. Dabei wird das Tiefziehmaterial durch den Materialhersteller, einen Materialtyp, eine Materialdicke und eine mechanische Materialeigenschaft beschrieben. Die mechanische Materialeigenschaft beschreibt dabei zum Beispiel einen Elastizitäts-Modul in Abhängigkeit einer Temperatur.

Basierend auf dem virtuellen Zahnmodell 28 und den Materialdaten D2 wird anschließend ein virtuelles Zahnschienenrohlingmodell 30 erzeugt. Hierzu wird basierend auf dem Zahnmodell 28 und den Materialdaten D2 eine Tiefziehsimulation ausgeführt. Im Rahmen dieser Tiefziehsimulation wird ein Tiefziehen des Tiefziehmaterials über das virtuelle Zahnmodell 28 nachgestellt.

Mit anderen Worten wird mittels einer Computersimulation, d. h. im virtuellen Raum, ermittelt, wie ein Zahnschienenrohling aussieht, der dadurch gebildet wird, dass das Tiefziehmaterial über das virtuelle Zahnmodell 28 tiefgezogen wird.

Ein solches virtuelles Zahnschienenrohlingmodell 30 ist in stark vereinfachter Weise in Figur 2 dargestellt. Dabei betrifft das virtuelle Zahnmodell 28, auf dem das virtuelle Zahnschienenrohlingmodell 30 platziert ist, einen Unterkiefer.

Das virtuelle Zahnschienenrohlingmodell 30 sowie ein zugehöriger, realer Zahnschienenrohling, umfasst somit eine Innenfläche 32, die dazu vorgesehen ist, an den Zähnen des Unterkiefers anzuliegen. Beim virtuellen Zahnschienenrohlingmodell 30 liegt die Innenfläche am virtuellen Zahnmodell 28 an.

Eine Außenfläche 34 des virtuellen Zahnschienenrohlingmodells 30 ist somit auf einer der Innenfläche abgewandten Seite angeordnet. Mit anderen Worten weist die Außenfläche 34 einen Abschnitt auf, der im Bereich der Kauflächen der Zähne des Zahnmodells liegt. Dieser Abschnitt wird auch als Aufbissfläche 36 bezeichnet. Für den realen Zahnschienenrohling gilt dasselbe.

Darüber hinaus umfasst die Außenfläche 34 einen lingualen Abschnitt 38, der die lingual orientierten Seitenflächen der Zähne des virtuellen Zahnmodells 28 bedeckt und einen labialen Abschnitt 40, der die labial orientierten Seitenflächen der Zähne des virtuellen Zahnmodells 28 bedeckt. Für den realen Zahnschienenrohling gilt wieder dasselbe.

Gemäß einer Alternative kann das virtuelle Zahnschienenrohlingmodell 30 erhalten werden, anstelle im Zuge des Verfahrens zum Bestimmen der Soll-Außenfläche 46 erzeugt zu werden.

Darüber hinaus wird im Zuge des Verfahrens ein virtuelles Antagonistenmodell 42 erhalten. Das Antagonistenmodell 42 umfasst wenigstens Modelle derjenigen Zähne, die den Zähnen gegenüber liegen, über welche sich die Zahnschiene 24 erstrecken soll.

Nachdem sich im dargestellten Ausführungsbeispiel die Zahnschiene 24 über alle Zähne des Unterkiefers erstrecken soll, umfasst das Antagonistenmodell 42 Repräsentationen aller Zähne des Oberkiefers. Das Antagonistenmodell 42 ist stark vereinfacht ebenfalls in Figur 2 dargestellt.

Das virtuelle Antagonistenmodell 42 ist, wie das virtuelle Zahnmodell 28, basierend auf patientenindividuellen Scan-Daten erstellt, die mittels eines Intraoralscans an demjenigen Patienten generiert wurden, für den die Zahnschiene 24 hergestellt werden soll.

Zusätzlich zur Geometrie der Zähne des Oberkiefers beschreibt das Antagonistenmodell 42 eine Beweglichkeit der Zähne des Antagonistenmodells 42 relativ zu den Zähnen des virtuellen Zahnmodells 28.

In der dargestellten Ausführungsform umfasst das Antagonistenmodell 42 dabei eine Beschreibung einer Bewegungskurve 44, entlang derer sich der Oberkiefer und der Unterkiefer relativ zueinander bewegen. Es versteht sich dabei, dass es im virtuellen Raum lediglich auf die Relativbewegung zwischen der Repräsentation des Oberkiefers und der Repräsentation des Unterkiefers ankommt. In der realen Welt kann der Oberkiefer als feststehend angenommen werden, sodass sich lediglich der Unterkiefer bewegt. Die Bewegungskurve 44 ist in Figur 2 stark vereinfacht eingezeichnet. Auch diese Bewegungskurve 44 ist anhand von Patientendaten ermittelt, d. h. die relative Beweglichkeit von Oberkiefer und Unterkiefer wurde an demjenigen Patienten gemessen, für den die Zahnschiene 24 vorgesehen ist. Basierend auf diesen Messungen wurde die Bewegungskurve 44 ermittelt.

Nun wird die Soll-Außenfläche 46 der Zahnschiene 24 basierend auf dem virtuellen Antagonistenmodell 42 und dem virtuellen Zahnschienenrohlingmodell 30 bestimmt.

Dabei wird im vorliegenden Beispiel angenommen, dass die Außenfläche 34 lediglich im Bereich der Aufbissfläche 36 von der Soll-Außenfläche 46 abweicht.

Das Bestimmen der Soll-Außenfläche 46 betrifft also das Ermitteln einer modifizierten Außenfläche im Bereich der Aufbissfläche 36. Dabei wird die Soll-Außenfläche 46 derart gewählt, dass eine Relativbewegung des Unterkiefers zum Oberkiefer, d. h. des Zahnmodells 28 zum Antagonistenmodell 42 möglichst wenig eingeschränkt ist. In diesem Zusammenhang ist insbesondere das Schließen des Gebisses relevant. Das bedeutet, dass die Soll-Außenfläche 46 derart bestimmt wird, dass der Unterkiefer, d. h. das virtuelle Zahnmodell 28, entlang der Bewegungskurve 44 insgesamt möglichst weit in Richtung zum Oberkiefer, d. h. in Richtung zum Antagonistenmodell 42 bewegt werden kann. Dabei wird ferner eine vorgegebene Mindestdicke d der Zahnschiene 24 berücksichtigt. Diese Mindestdicke d darf nicht unterschritten werden, um ein vorgegebenes Mindestmaß an mechanischer Stabilität der Zahnschiene zu gewährleisten.

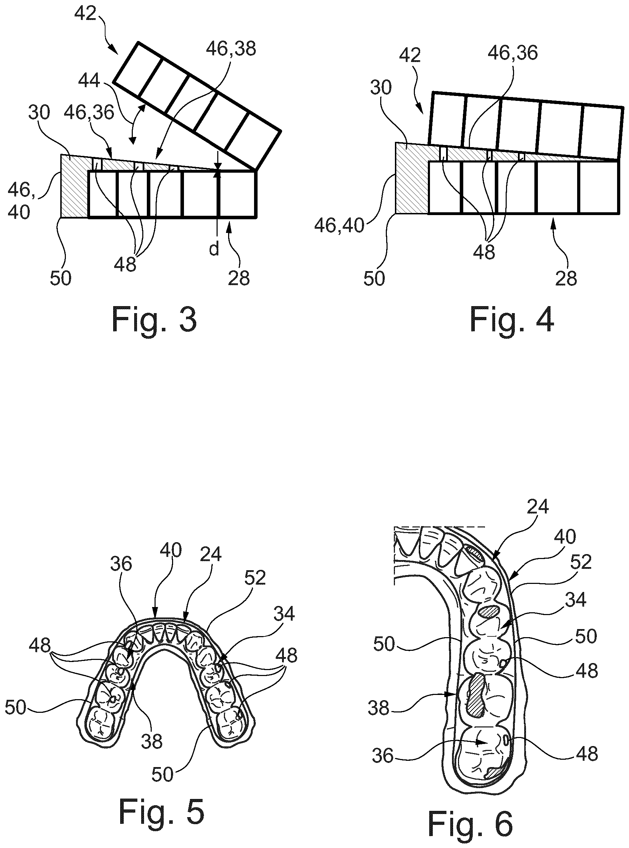

Die Soll-Außenfläche 46 ist in Figur 3 in stark vereinfachter Form visualisiert. Dabei ist im Bereich des hintersten Backenzahns die vorgegebene Mindestdicke d der Zahnschiene erreicht. Aufgrund der Beweglichkeit des Oberkiefers und des Unterkiefers relativ zueinander entlang der Bewegungskurve 44 ergibt sich eine Art Keilform der Zahnschiene.

Die durch das Ermitteln der Soll-Außenfläche 46 verbesserte Beweglichkeit des Unterkiefers und des Oberkiefers, d. h. des virtuellen Zahnmodells 28 und des Antagonistenmodells 42, relativ zueinander lässt sich besonders gut anhand einer Zusammenschau der Figuren 2 und 4 erkennen. In beiden Figuren ist das virtuelle Gebiss, das das virtuelle Zahnmodell 28 und das virtuelle Antagonistenmodell 42 umfasst, maximal geschlossen.

Gemäß einer Option können im Zuge des Verfahrens zum Bestimmen der Soll-Außenfläche auch eine oder mehrere Durchgangsöffnungen 48 für Speichel und/oder Nahrungspartikel virtuell positioniert werden.

Einige wenige solche Durchgangsöffnungen 48 sind dabei schematisch in den Figuren 3 und 4 eingezeichnet. Diese sind als rein illustrativ zu verstehen.

Gemäß einer weiteren Option kann im Zuge des Verfahrens zum Bestimmen der Soll-Außenfläche 46 auch ein Saum 50 der Zahnschiene basierend auf dem virtuellen Zahnmodell 28 und basierend auf dem virtuellen Zahnschienenrohlingmodell 30 virtuell positioniert werden. Das bedeutet, dass im Zuge des Verfahrens zum Bestimmen der Soll-Außenfläche festgelegt wird, entlang welcher Linie die Zahnschiene nach dem Tiefziehen und spanenden Bearbeiten aus dem restlichen Tiefziehmaterial herausgeschnitten oder herausgefräst wird.

Nun kann die Zahnschiene 24 basierend auf den Ergebnissen des Verfahrens zum Bestimmen der Soll-Außenfläche hergestellt werden.

Wie bereits erwähnt, wird dabei im vorliegenden Ausführungsbeispiel zum Herstellen der Zahnschiene 24 ein Tiefziehverfahren verwendet. Dabei wird ein folienförmiges Tiefziehmaterial über ein physisches Zahnmodell, das 3D-gedruckt sein kann, tiefgezogen.

Das physische Zahnmodell kann dann inklusive der darüber tiefgezogenen Folie in die Herstellungsvorrichtung 22, d. h. in die Fräsmaschine eingesetzt werden.

Mit anderen Worten ist die herzustellende Zahnschiene 24 in demjenigen Zwischenzustand, in dem sie von der Herstellungsvorrichtung 22 weiterverarbeitet wird, auf einem zugehörigen Tiefziehwerkzeug 52 gelagert. Das ist selbstverständlich nur eine Möglichkeit. Alternativ dazu kann die tiefgezogene Zahnschiene in diesem Zwischenzustand auch auf ein anderes, vom Tiefziehwerkzeug 52 separates Werkzeug umgelagert werden.

Die Ergebnisse des Verfahrens zum Bestimmen der Soll-Außenfläche der Zahnschiene 24 werden dann von der Fräsmaschine, d. h. von der Herstellungsvorrichtung 22 in Form von Außenflächendaten D1 erhalten.

In der dargestellten Ausführungsform beschreiben die Außenflächendaten D1 die Soll-Außenfläche 46 der Zahnschiene sowie optional die Durchgangsöffnungen 48 sowie den Saum 50.

Nun kann mittels der Herstellungsvorrichtung 22 eine Ist-Außenfläche der Zahnschiene 24 in ihrem aktuellen Zwischenzustand an die Soll-Außenfläche 46 angeglichen werden. Konkret bedeutet das, dass die Außenfläche 34 der Zahnschiene 24 im Bereich der Aufbissfläche 36 derart abgefräst wird, dass die Ist-Außenfläche der Soll-Außenfläche 46 entspricht.

Darüber hinaus können optional mittels der Herstellungsvorrichtung 22, d. h. mittels der Fräsmaschine, die bereits virtuell positionierten Durchgangsöffnungen 48 durch spanendes Bearbeiten gefertigt werden. Ebenfalls kann der Saum der Zahnschiene 24, der ebenfalls bereits virtuell positioniert wurde, gefräst werden.

Gemäß einer weiteren Option kann die Zahnschiene 24 mittels der Herstellungsvorrichtung 22, d. h. mittels der Fräsmaschine poliert werden.

Die Zahnschiene 24 ist somit durch Anwendung des Verfahrens zum Bestimmen der Soll-Außenfläche und des Verfahrens zum Herstellen der Zahnschiene 24 im Wesentlichen in automatisierter Weise hergestellt.

Neben den stark vereinfachten Darstellungen aus den Figuren 2 bis 4 zeigt in diesem Zusammenhang die Figur 5 die Zahnschiene 24 in einem Zustand direkt nach dem Tiefziehen, d. h. vor einem Angleichen der Ist-Außenfläche an die Soll-Außenfläche 46.

Figur 6 zeigt die Zahnschiene 24 in einem nächsten Zwischenzustand, d. h. in einem Zwischenzustand, in dem ein Teil der Ist-Außenfläche bereits an die Soll-Außenfläche 46 durch spanende Bearbeitung, d. h. durch Fräsen, angeglichen wurde. Um dies zu illustrieren, sind in der Figur 6 einige Bereiche der Außenfläche der Zahnschiene 24 mit einer Schraffur versehen. Diese Schraffuren kennzeichnen Abschnitte der Zahnschiene 24, in denen ein Angleichen der Ist-Außenfläche an die Soll-Außenfläche 46 nötig war. Vereinfacht gesagt, werden durch die Schraffuren Bereiche der Zahnschiene 24 markiert, die zum Angleichen der Ist-Außenfläche an die Soll-Außenfläche 46 abgefräst wurden. Es versteht sich, dass die schraffierten Bereiche in Figur 6 rein illustrativ sind und nicht zwingend realitätsgetreu.

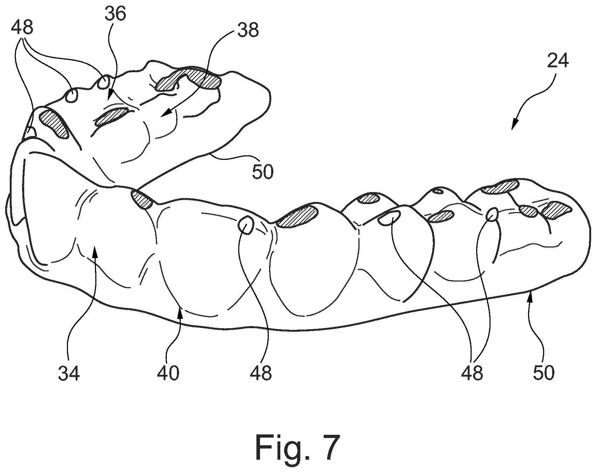

Die fertig hergestellte Zahnschiene ist in Figur 7 separat dargestellt. Wieder sind diejenigen Bereiche der Außenfläche der Zahnschiene 24, in denen ein Angleichen der Ist-Außenfläche an die Soll-Außenfläche 46 stattgefunden hat, mit einer Schraffur versehen. Dies ist wieder rein illustrativ, d. h. nicht zwingend realitätsgetreu.

### Bezugszeichenliste

- 10: Vorrichtung zur Datenverarbeitung
- 12: Speichereinheit
- 14: Recheneinheit
- 16: computerlesbaren Medium
- 18: Computerprogramm zum Bestimmen der Soll-Außenfläche
- 20: Mittel zum Ausführen des Verfahrens zum Bestimmen einer Soll-Außenfläche einer Zahnschiene
- 22: Herstellungsvorrichtung
- 24: Zahnschiene
- 26: Computerprogramm zum Ansteuern der Herstellungsvorrichtung
- 28: virtuelles Zahnmodell
- 30: virtuelles Zahnschienenrohlingmodell
- 32: Innenfläche
- 34: Außenfläche
- 36: Aufbissfläche
- 38: lingualer Abschnitt
- 40: labialer Abschnitt
- 42: Antagonistenmodell
- 44: Bewegungskurve
- 46: Soll-Außenfläche
- 48: Durchgangsöffnung
- 50: Saum
- 52: Tiefziehwerkzeug

- d: Mindestdicke
- D1: Außenflächendaten
- D2: Materialdaten

## Patentansprüche

1. Computer-implementiertes Verfahren zum Bestimmen einer Soll-Außenfläche (46) einer Zahnschiene (24), umfassend
- Erzeugen eines virtuellen Zahnschienenrohlingmodells (30) basierend auf einem virtuellen Zahnmodell (28), welches zumindest virtuelle Teil-Modelle derjenigen Zähne umfasst, über die sich die Zahnschiene (24) erstrecken soll, und basierend auf Materialdaten (D2), die wenigstens eine Eigenschaft eines Materials beschreiben, aus dem die Zahnschiene (24) hergestellt werden soll, oder
Erhalten eines virtuellen Zahnschienenrohlingmodells (30),
- Erhalten eines virtuellen Antagonistenmodells (42), welches zumindest virtuelle Teil-Modelle derjenigen Zähne umfasst, die den Zähnen gegenüberliegen, über die sich die Zahnschiene (24) erstrecken soll, und
- Bestimmen der Soll-Außenfläche (46) der Zahnschiene (24) basierend auf dem virtuellen Zahnschienenrohlingmodell (30) und dem virtuellen Antagonistenmodell (42).

2. Verfahren nach Anspruch 1, wobei die Soll-Außenfläche (46) eine Aufbissfläche (36) umfasst.

3. Verfahren nach Anspruch 1 oder 2, wobei das virtuelle Antagonistenmodell (42) ferner eine Beweglichkeit der Zähne des Antagonistenmodells (42) relativ zu den Zähnen des virtuellen Zahnmodells (28) beschreibt.

4. Verfahren nach Anspruch 3, ferner umfassend
Erhalten von Patientendaten, welche die Beweglichkeit der Zähne des Antagonistenmodells (42) relativ zu den Zähnen des virtuellen Zahnmodells (28) beschreiben.

5. Verfahren nach einem der vorhergehenden Ansprüche, wobei die Materialdaten (D2) ein Tiefziehmaterial beschreiben und wobei das Erzeugen eines virtuellen Zahnschienenrohlingmodells (30) ein Ausführen einer Tiefziehsimulation umfasst, bei der ein Tiefziehen des Tiefziehmaterials über das Zahnmodell (28) nachgestellt wird.

6. Verfahren nach einem der vorhergehenden Ansprüche, wobei eine Eigenschaft des Materials einen Materialhersteller, einen Materialtyp, eine Materialdicke und/oder eine mechanische Materialeigenschaft beschreibt.

7. Verfahren nach einem der vorhergehenden Ansprüche, ferner umfassend virtuelles Positionieren von einer oder mehreren Durchgangsöffnungen (48) in der Zahnschiene (24) basierend auf dem virtuellen Zahnmodell (28) und/oder virtuelles Positionieren wenigstens eines Abschnitts eines Saumes (50) der Zahnschiene (24) basierend auf dem virtuellen Zahnmodell (28).

8. Verfahren nach einem der vorhergehenden Ansprüche, wobei beim Bestimmen der Soll-Außenfläche (46) der Zahnschiene (24) basierend auf dem virtuellen Zahnschienenrohlingmodell (30) und dem virtuellen Antagonistenmodell (42) ferner eine vorgegebene Mindestdicke der Zahnschiene (24) berücksichtigt wird.

9. Verfahren zum Herstellen einer Zahnschiene (24), umfassend
- Erhalten von Außenflächendaten (D1), die zumindest einen Abschnitt einer Soll-Außenfläche (46) der Zahnschiene (24) beschreiben, insbesondere wobei die Außenflächendaten (D1) mittels eines Verfahrens nach einem der vorhergehenden Ansprüche bestimmt sind, und
- Angleichen einer Ist-Außenfläche der Zahnschiene (24) an die Soll-Außenfläche (46) durch spanendes Bearbeiten.

10. Verfahren nach Anspruch 9, wobei das Angleichen der Ist-Außenfläche an die Soll-Außenfläche (46) erfolgt, während die Zahnschiene (24) auf einem Tiefziehwerkzeug (52) gelagert ist oder während die Zahnschiene (24) auf einer vom Tiefziehwerkzeug (52) separaten Vorrichtung gelagert ist.

11. Verfahren nach Anspruch 9 oder 10, ferner umfassend
Einbringen von einer oder mehreren Durchgangsöffnungen (48) durch spanendes Bearbeiten und/oder
Erstellen wenigstens eines Abschnitts eines Saumes (50) der Zahnschiene (24) durch spanendes Bearbeiten, und/oder
zumindest abschnittsweises Polieren der Zahnschiene (24).

12. Zahnschiene (24) erhalten mittels eines Verfahrens nach einem der Ansprüche 9 bis 11.

13. Vorrichtung (10) zur Datenverarbeitung, umfassend Mittel (20) zur Ausführung des Verfahrens nach einem der Ansprüche 1 bis 8.

14. Computerprogramm (18), umfassend Befehle, die bei der Ausführung des Computerprogramms (18) durch einen Computer diesen veranlassen, das Verfahren nach einem der Ansprüche 1 bis 8 auszuführen.

15. Computerlesbares Medium (16), umfassend Befehle, die bei der Ausführung durch einen Computer diesen veranlassen, das Verfahren nach einem der Ansprüche 1 bis 8 auszuführen.
